(19) Europäisches Patentamt
European Patent Office
Office européen des brevets

(11) Veröffentlichungsnummer: **0 249 977**
**B1**

# (12) EUROPÄISCHE PATENTSCHRIFT

(45) Veröffentlichungstag der Patentschrift: **31.10.90**

(51) Int. Cl.[5]: **C07D 231/16**, C07D 231/12, A01N 43/50, A61K 31/415

(21) Anmeldenummer: **87108760.7**

(22) Anmeldetag: **19.06.87**

(54) **Hydroxipyrazol-Derivate- Verfahren zu ihrer Herstellung und ihre Verwendung gegen Mikroorganismen.**

(30) Priorität: **19.06.86 DE 3620579**

(43) Veröffentlichungstag der Anmeldung: **23.12.87 Patentblatt 87/52**

(45) Bekanntmachung des Hinweises auf die Patenterteilung: **31.10.90 Patentblatt 90/44**

(84) Benannte Vertragsstaaten: **AT BE CH DE ES FR GB GR IT LI NL SE**

(56) Entgegenhaltungen:
**DE-A- 3 409 317**
**DE-A- 3 532 880**

(73) Patentinhaber: **BASF Aktiengesellschaft, Carl-Bosch-Strasse 38, D-6700 Ludwigshafen(DE)**

(72) Erfinder: **Wagner, Klaus, Dr., Sauterstrasse 32, D-6730 Neustadt(DE)**
Erfinder: **Rieber, Norbert, Dr., Liebfrauenstrasse 1 c, D-6800 Mannheim 51(DE)**
Erfinder: **Pommer, Ernst-Heinrich, Dr, Berliner Platz 7, D-7603 Limburgerhof(DE)**

**EP 0 249 977 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue, wertvolle Hydroxipyrazol-Derivate mit fungizider, bakterizider und algizider Wirksamkeit, Verfahren zu ihrer Herstellung, Mikrozide, die diese Verbindungen als Wirkstoffe enthalten und Verfahren zur Bekämpfung von Pilzen, Bakterien und Algen (Mikroorganismen).

Es ist bekannt, N-Trichlormethylthio-phthalimid (Chemical Week 1972, June 21, Seite 63), Tetramethylthiuramdisulfid (Chemical Week 1972, July 26, Seite 39) oder 2-Thiocyanomethylthio-benzothiazol (Farm Chemicals Handbook 1976, Seite D 43) als Fungizide bzw. Bakterizide zu verwenden. Ihre Wirkung ist jedoch unbefriedigend. Es sind ferner die Alkyl-O- und CN-Alkyl-O-substituierten Derivate das halogensubstituierten 1-Hydroxipyrazols bekannt (DE-A-34 09 317), z.B. das 4-Chlor-1-methoxypyrazol und das 4-Chlor-1-cyanomethoxy-pyrazol.

Es wurde gefunden, daß 1-Hydroxipyrazol-Derivate der Formel I

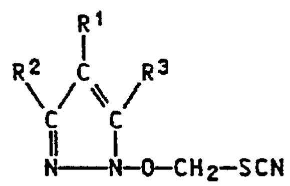

I

in der $R^1$, $R^2$ und $R^3$ unabhängig voneinander Wasserstoff oder Halogen (Chlor, Brom oder Jod) bedeuten, eine ausgezeichnete mikrobiozide Wirksamkeit aufweisen und wirksamer sind als die bekannten Wirkstoffe. Als besonders bevorzugte Verbindung ist 4-Halogen-1-[(thiocyanato)methoxy]-pyrazol, insbesondere 4-Chlor-1-[(thiocyanato)-methoxy]-pyrazol zu nennen.

Die erfindungsgemäßen Verbindungen werden durch Umsetzung von 1-Hydroxipyrazolen der Formel II

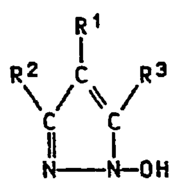

II,

in der $R^1$, $R^2$ und $R^3$ die oben angegebene Bedeutung haben, mit den Thiocyanaten der Formel III

$R^4$–$CH_2$–SCN (III),

in der $R^4$ Halogen (Chlor, Brom oder Jod) bedeutet, erhalten.

Verbindungen der Formel II werden in der DE-OS 3 205 456 beschrieben. Alkyl-O- und CN-Alkyl-O-substituierte Derivate von 1-Hydroxipyrazol mit nitrifikationsinhibierender Wirkung in der DE-OS 3 409 317 beschrieben. Die Verbindungen der Formel III sind ebenfalls bekannt (DE-OS 2 648 965, EP 65 190).

Zur Herstellung der erfindungsgemäßen Verbindungen läßt man die Reaktionspartner vorzugsweise in inerten Lösungsmitteln, wie aliphatischen und aromatischen, gegebenenfalls chlorierten Kohlenwasserstoffen, z.B. Toluol oder Methylenchlorid, Ethern, z.B. Diethylether oder Tetrahydrofuran, Alkoholen, z.B. tert.-Butanol, Ketonen, z.B. Aceton, Nitrilen, z.B. Acetonitril, oder Amiden, z.B. N,N-Dimethylformamid, in Anwesenheit einer Base, z.B. eines tertiären Amins, Pyridins, Alkalicarbonats oder Alkalialkoholats bzw. -hydrids, zwischen –60 und 180°C, vorzugsweise zwischen 20 und 80°C, miteinander reagieren. Die Durchführung der genannten Reaktion ist auch im Zwei-Phasen-System unter Phasentransfer-Katalyse möglich. Dazu werden bevorzugt chlorierte Kohlenwasserstoffe, z.B. Methylenchlorid, wäßrige Laugen, z.B. Natronlauge und ein Phasentransfer-Katalysator, z.B. Tetra-n-butylammoniumhydroxid, bei Temperaturen von 10°C bis zur Rückflußtemperatur der Mischung der Reaktionspartner eingesetzt.

<u>Beispiel 1</u>

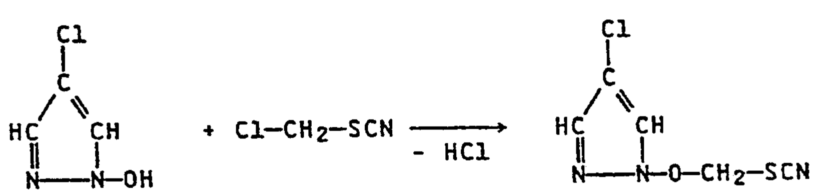

Zu 1,4 g (35 mMol) Kaliumhydrid in 100 ml trockenem Tetrahydrofuran tropfte man bei 10 bis 15°C 2,5 g (21 mMol) 4-Chlor-1-hydroxipyrazol in 50 ml trockenem Tetrahydrofuran. Anschließend wurde 30 min. bei Raumtemperatur nachgerührt. Nach dem Abkühlen auf 15°C wurden 4,5 g (21 mMol) Chlormethylthiocyanat in 20 ml trockenem Tetrahydrofuran langsam zugetropft und bei Raumtemperatur 5 Tage nachgerührt. Das Reaktionsgemisch wurde dann zwischen dann zwischen Wasser und Methylenchlorid verteilt, die organische Phase mit Natriumcarbonatlösung extrahiert, neutralgewaschen und über Magnesiumsulfat getrocknet. Nach dem Abziehen des Lösungsmittels im Rotationsverdampfer destillierte man aus dem Rückstand zunächst das überschüssige Chlormethylthiocyanat (Luftbad bis 90°C) und anschließend (Luftbad bis 160°C/2 mbar) 3,1 g (78 % d.Th.) 4-Chlor-1-[(thiocyanato)-methoxy]-pyrazol (Verbindung Nr. 1) über, das nach der Kristallisation aus Ether/Petrolether einen Fp. von 60°C aufwies.

Beispiel 2

5 g (42 mMol) 4-Chlor-1-hydroxipyrazol, 13,5 g (126 mMol) Chlormethylthiocyanat und 50 ml Methylenchlorid versetzte man mit 50 ml einer 5 %igen wäßrigen Natronlaugelösung. Nach der Zugabe von 1 g (4 mMol) Tetra-n-butylammoniumhydroxid rührte man 24 h bei Raumtemperatur. Anschließend wurde die organische Phase abgetrennt, getrocknet und eingedampft. Durch Destillation des Rückstandes im Vakuum (Luftbad bis 140°C/3 mbar) erhielt man 5,7 g (71 % d.Th.) 4-Chlor-1-[(thiocyanato)-methoxy]-pyrazol (Verbindung Nr. 1), das aus Ether/Petrolether umkristallisiert wurde (Fp. 60°C).

Beispiel 3

42 g (1,4 Mol) 80 %iges (Gew.%) Natriumhydrid wurden in 1000 ml trockenem Tetrahydrofuran (THF) vorgelegt. Unter gelinder Kühlung tropfte man dazu 100 g (0,84 Mol) 4-Chlor-1-hydroxipyrazol gelöst in 200 ml trockenem THF. Nachdem man 0,5 h nachgerührt hatte, tropfte man bei 15°C 180,6 g (1,68 Mol) Chlormethylthiocyanat in 200 ml trockenem THF zu und ließ 120 h bei Raumtemperatur (20°C) rühren. Anschließend wurde analog zu Beispiel 1 aufgearbeitet. Das Rohprodukt, das nach dem Entfernen des Lösungsmittels erhalten wurde, unterwarf man einer fraktionierten Destillation. Über eine kurze Destillationskolonne (15 cm) erhielt man bei 90°C/36 mbar 47,5 g Chlormethylthiocyanat zurück. Bei 90 bis 122°C/2 mbar destillierte man schließlich 125,9 g (78 % d.Th.) der Verbindung 1 ab.

Beispiel 4

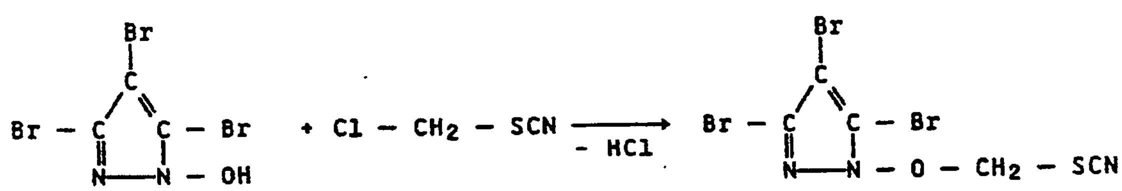


Man legte 2,8 g Kaliumhydrid in 200 ml trockenem THF vor und tropfte 13,5 g (42 mMol) 3,4,5-Tribrom-1-hydroxipyrazol in 50 ml trockenem THF hinzu. Nach 0,5 h Nachrühren tropfte man bei 15°C 9,0 g (84 mMol) Chlormethylthiocyanat in 40 ml trockenem THF zu und rührte weitere 120 h. Dann arbeitete man auf wie in Beispiel 1 beschrieben und erhielt so 10,2 g (61 % d.Th.) von Verbindung 4, die abschließend an Kieselgel (Ether/Petrolether = 1:1) chromatographiert wurde.

Durch entsprechende Abwandlungen des Herstellverfahrens lassen sich beispielsweise 3,4-Dichlor-, 3,4,5-Trichlor-, 3-Brom-4-chlor, 4-Chlor-3-iod-, 4-Chlor-3,5-dibrom-, 4-Chlor-3,5-diiod-, 4-Brom-3-chlor-, 4-Brom-3-iod-, 4-Brom-3,5-dichlor-, 4-Brom-3,5-diiod-, 3,4-Diiod-, 3,4,5-Triiod-, 3-Brom-4-iod-, 3-Chlor-4-iod-, 4-Iod-3,5-dibrom- und 4-Iod-3,5-dichlor-1-[thiocyanato-methoxi]pyrazol gewinnen.

| Verbindung | $R^1$ | $R^2$ | $R^3$ | Fp. [°C] | $^1H$-NMR ($CDCl_3$) |
|---|---|---|---|---|---|
| 1 | Cl | H | H | 60 | 7,45 (s, 1H), 7,20 (s, 1H), 5,70 (s, 2H) |
| 2 | Br | H | H | 60 | 7,50 (s, 1H), 7,30 (s, 1H), 5,75 (s, 2H) |
| 3 | Br | Br | H | 81 | 7,50 (s, 1H), 5,75 (s, 2H) |
| 4 | Br | Br | Br | 103 | 5,80 (s) |
| 5 | J | H | H | 103 | 7,55 (s, 1H), 7,45 (s, 1H), 5,80 (s, 2H) |
| 6 | H | H | H | Öl | 7,45 (m, 1H), 7,30 (m, 1H), 6,20 (t, 1H), 5,80 (s, 2H) |

Die neuen Wirkstoffe eignen sich insbesondere zum Schutz von verschiedenen Materialien gegen den Abbau bzw. die Zerstörung durch Bakterien oder Pilze oder gegen den Befall und Bewuchs durch Mikroorganismen. Materialien, die mit den neuen Wirkstoffen konserviert bzw. mikrozid ausgerüstet werden können, sind beispielsweise Leime und Klebstoffe, Stärkelösungen, Wachsemulsionen, Tonemulsionen, Schlichten, Appreturen, Spinnbäder, Gelatinezubereitungen, Fensterkitt, Fugendichtungsmassen, Kühlschmierstoffe, Bohröle, Treibstoffe, Kunststoffdispersionen, Dispersionsfarben, Textilien, Leder, Rohhäute, Kosmetika. Weiterhin sind die Verbindungen als Schleimbekämpfungsmittel in der Papierindustrie, in Rückkühlwerken und in Luftbefeuchtungsanlagen geeignet.

Folgende Mikroorganismen lassen sich beispielsweise mit den erfindungsgemäßen Verbindungen bekämpfen: Staphylococcus aureus, Escherichia coli, Klebsiella pneumoniae, Citrobacter freundii, Proteus vulgaris, Pseudomonas aeruginosa, Desulfovibrio desulfuricans, Streptoverticillium rubrireticuli, Aspergillus niger, Aspergillus versicolor, Penicillium funiculosum, Penicillium expansum, Penicillium glaucum, Peacilomyces varioti, Trichoderma viride, Chaetomium globosum, Aspergillus amstelodami, Phoma pigmentovora, Phoma violacea, Aureobasidium pullulans, Saccharomyces cerevisiae, Alternaria tenuis, Stemphylium macrosporoideum, Cladosporium herbarum, Cladosporium resinae, Candida albicans, Trichophyton mentagrophytes, Geotrichum candidans, Monilia sitophila, Scenedesmus quadricauda, Chlorella vulgaris, Nostoc muscorium, Oscillatoria limosa und Anabaena constricta.

Die neuen Wirkstoffe werden in Form von Zubereitungen angewendet. Gegenstand der vorliegenden Erfindung sind demnach auch Mittel oder Zubereitungen, die neben üblichen Verdünnungsmitteln und Trägern eine Verbindung der Formel I enthalten. Die Zubereitungen, wie Lösungen, Emulsionen, Suspensionen, Pulver und Pasten, werden in bekannter Weise angewendet. Die mikroziden Mittel enthalten beispielsweise 0,5 bis 95 % (Gew.%) an Wirkstoff. Als übliche Anwendungskonzentration wählt man - bezogen auf das Gewicht des zu schützenden Materials - 0,001 bis 5 % an Wirkstoff; beim Einsatz zur Wasserbehandlung, bei der Erdölförderung, in Bohr- und Schneidölen, Treibstoffen, Rückkühlwerken, Luftbefeuchtungsanlagen oder in der Papierindustrie sind Wirkstoffmengen von 5 bis 500 ppm ausreichend. Die Wirkstoffe können auch mit anderen bekannten Mikroziden gemischt erden. In vielen Fällen erhält man dabei einen synergistischen Effekt. Die folgende Liste von Bakteriziden und Fungiziden, mit denen die erfindungsgemäßen Verbindungen kombiniert werden können, soll die Mischungsmöglichkeiten aufzeigen, nicht aber einschränken. Durch die Kombination mit anderen Wirkstoffen erhält man in vielen Fällen eine Vergrößerung des mikroziden Wirkungsspektrums; bei einer Anzahl dieser Mikrozidmischungen treten auch synergistische Effekte auf, d.h. die mikrozide Wirksamkeit des Kombinationsproduktes ist größer als die der addierten Wirksamkeiten der Einzelkomponenten. Die Zumischung dieser Wirkstoffe zu den erfindungsgemäßen Verbindungen kann im Gewichtsverhältnis 1:1 bis 100:1 erfolgen. Solche Wirkstoffe sind beispielsweise: 2-(Thiocyanomethylthio)-benzthiazol
1-2-(2,4-Dichlorphenyl)-2-(2-propenyl-oxy)ethyl-1H-imidazol
2,4,5,6-Tetrachlor-isophthalodinitril
Methylenbisthiocyanat
Tributylzinnoxid, -chlorid, -naphthenat, -benzoat, -salicylat
Mercaptobenzthiazol
1,2-Benzisothiazolon und seine Alkalisalze
Alkaliverbindungen des N'-Hydroxy-N-cyclohexyl-idazeniumoxids
2-(Methoxy-carbonylamino)-benzimidazol
3-Methyl-3-oxo-5-chlor-thiazolin-3-on

Trihydroxymethyl-nitro-methan
Glutardialdehyd
Chloracetamid
Polyhexamethylenbisguanide
5-Chlor-2-methyl-4-isothiazolin-3-on + Magnesiumsalze
3,5-Dimethyltetrahydro-1,3,5-2H-thiadiazin-2-thion
Hexahydrotriazin
N,N-Methylolchloracetamid
2-n-Octyl-4-isothiazol-in-3-on
Oxazolidine
Bisoxaolidine
2,5-Dihydro-2,5-dialkoxy-2,5-dialkylfurane
Diethyl-dodecyl-benzyl-ammoniumchlorid
Dimethyl-octadecyl-dimethylbenzyl-ammoniumchlorid
Dimethyl-didecyl-ammoniumchlorid
Dimethyl-didodecyl-ammoniumchlorid
Trimethyl-tetradecylammoniumchlorid
Benzyl-dimethyl-alkyl-($C_{12}$-$C_{18}$)-ammoniumchlorid
Dichlorbenzyl-dimethyl-dodecyl-ammoniumchlorid
Cetylpyridiniumchlorid
Cetylpyridiniumbromid
Cetyl-trimethyl-ammoniumchlorid
Laurylpyridniumchlorid
Laurylpyridniumbisulfat
Benzyl-dodecyl-di(β-oxethyl)-ammoniumchlorid
Dodecylbenzyl-trimethyl-ammoniumchlorid
n-Alky-dimethyl-benzyl-ammoniumchlorid
(Alkylrest: 40 % $C_{12}$, 50 % $C_{14}$, 10 % $C_{16}$)
Lauryl-dimethyl-ethyl-ammoniumethylsulfat
n-Alkyl-dimethyl-(1-naphthylmethyl)-ammoniumchlorid
(Alkylrest: 98 % $C_{12}$, 2 % $C_{14}$)
Cetyldimethylbenzylammoniumchlorid
Lauryldimethylbenzylammoniumchlorid

Mischungspartner 1,3-Dimethylol-5,5-dimethylhydantoin
Dimethylolharnstoff
Tetramethylolacetylendiharnstoff
Dimethylolglyoxalmonourein
Hexamethylentetramin
Glyoxal
Glutardialdehyd
N-Methylol-chloracetamid
1-(Hydroxymethyl)-5,5-dimethyl-hydantoin
1,3-Bis-(hydroxymethyl)-5,5-dimethylhydantoin
Imidazolidinylharnstoff
1-(3-Chlorallyl)-3,5,7-triaza-1-azonia-adamantan-chlorid
1,3-Bis-(β-ethylhexyl)-5-methyl-5-amino-hexahydropyrimidin
1,3,5-Tris-(hydroxyethyl)-1,3,5-hexahydrotriazin
1,2-Dibrom-2,4-dicyanobutan
5-Brom-5-nitro-1,3-dioxan
2-Brom-2-nitropropandiol
1,1'-Hexamethylen-bis-[5-(4-chlorphenyl)-biguanid]
4,4-Diaminodiphenoxypropan
2-Brom-2-nitro-propan-1,3-diol
Sorbinsäure und ihre Salze
p-Hydroxybenzoesäure und ihre Ester und Salze
Zink-2-pyridinethiol-N-oxid
2-[(Hydroxylmethyl)amino]-ethanol
Dithio-2,2'-bis(benzmethyl-amid)
5-Chlor-2-(2,4-dichlorphenoxy)-phenol
Thio-bis-(4-chlorphenol)
o-Phenyl-phenol
Chlormethyl-dijodmethylsulfon
p-Chlorphenyl-3-jodpropargyl-formal

In den Anwendungsbeispielen werden die Wirksamkeiten von erfindungsgemäßen Verbindungen dargelegt. Folgende bekannte Wirkstoffe wurden zu Vergleichszwecken verwendet:

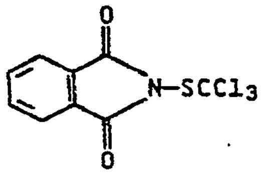

Wirkstoff A

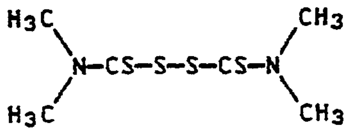

Wirkstoff B

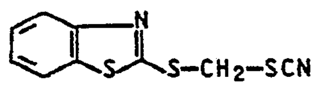

Wirkstoff C

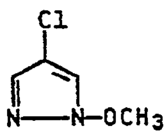

Wirkstoff D

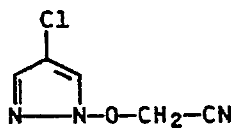

Wirkstoff E

Anwendungsbeispiel 1

Fungizide Wirksamkeit gegenüber Aspergillus niger

Die Wirkstoffe werden einer für das Wachstum des Pilzes Aspergillus niger optimal geeigneten Nährlösung in Mengen von 100, 75, 50, 25, 10, 5 und 2,5 Gewichtsteilen pro Million Teile Nährlösung zugesetzt. Es werden jeweils 20 ml der so behandelten Nährlösung in 100 ml Glaskolben mit 0,3 mg Aspergillus-Pilzsporen beimpft. Die Kolben werden 120 Stunden lang bei 36°C erwärmt und anschließend das Ausmaß der Pilzentwicklung, das bevorzugt auf der Nährlösungsoberfläche erfolgt, beurteilt.

Das Ergebnis des Versuches zeigt, daß beispielsweise die Verbindung 1 bei der Anwendung in der Verdünnung 5 zu 1 Million Gewichtsteile eine sehr gute fungizide Wirkung zeigt (100 %) während die bekannten Wirkstoffe A, B, C bei dieser Verdünnung keine Wirkung zeigen (0 %).

Anwendungsbeispiel 2

Wirksamkeit gegenüber den Pilzen Paecilomyces varioti, Aureobasidium pullulans, Geotrichum candidans

Zur Prüfung der Wirksamkeit gegenüber Pilzen werden die Wirkstoffe einer für das Wachstum der Pilze Paecilomyces variotii, Aureobasidium pullulans und Geotrichum candidans optimal geeigneten Nährlösung in Mengen von 100, 50, 25, 12, 6, 3 und 1,5 Gewichtsteilen pro Million Teile Nährlösung zugesetzt. Jeweils 10 ml Nährlösungs-Wirkstoffgemisch werden in sterile Reagenzgläser gegeben und mit einem Tropfen einer Sporensuspension beimpft, die $10^6$ Konidien bzw. Zellen enthält. Nach 120-stündiger Bebrütung werden aus denjenigen Röhrchen, die kein sichtbares Pilzwachstum zeigen, Proben entnommen und auf Pilznährböden übertragen. In der Tabelle wird die Verdünnungsstufe angegeben, bei welcher nach dem Übertragen einer Probe auf den Nährboden kein Wachstum der Pilze mehr erfolgt.

Das Ergebnis des Versuches zeigt, daß beispielsweise die Verbindungen 1, 2, 3, 4 und 6 bei der Anwendung von 6 ppm eine gute fungizide Wirkung haben, während die gleiche Wirkung mit den Wirkstoffen A, B, C, D und E erst bei 50 ppm erzielt wird.

Anwendungsbeispiel 3

Bakterizide Wirksamkeit gegenüber Staphylococcus aureus, Escherichia coli, Proteus vulgaris und Pseudomonas aeruginosa

Die Abtötungswerte gegenüber Bakterien wurden wie folgt ermittelt: Zu je 5 ml einer Verdünnung der Mittel in Wasser wurden 5 ml doppeltkonzentrierter Nährbouillon in sterile Reagenzgläser gegeben und vermischt. Die Versuchansätze enthalten 200, 100, 50, 25, 12, 6 und 3 Gewichtsteile Wirkstoff pro Million Teile Nährbouillon. Durch Zugabe von einem Tropfen 1:10 verdünnter 16 Stunden alter Bouillon-Kulturen der Bakterienarten Staphylococcus aureus, Escherichia coli, Proteus vulgaris bzw. Pseudomonas aeruginosa wurden dann die Röhrchen beimpft und 24 Stunden lang bei 37°C bebrütet. Nach dieser Zeit wurden Proben aus den Röhrchen auf Bakteriennährböden übertragen und diese ebenfalls 24 Stunden lang bei 37°C bebrütet. Diejenige Verdünnungsstufe, bei welcher nach dem Übertragen einer Probe auf den Nährboden keine Bakterienentwicklung mehr erfolgte, ist als Abtötungswert angegeben.

Das Ergebnis des Versuches zeigt, daß beispielsweise die Verbindungen 1, 2, 3, 4 und 6 bei der Anwendung von 25 ppm eine gute bakterizide Wirkung haben, während die Wirkstoffe C, D und E diese Wirkung erst bei 100 ppm haben.

Anwendungsbeispiel 4

Algizide Wirksamkeit gegenüber Grünalgen

Zur Prüfung der Wirksamkeit gegenüber Grünalgen werden die Wirkstoffe einer für die Vermehrung der einzelligen Grünalge Chlorella vulgaris begünstigenden, phosphatreichen Nährlösung in Mengen von 10, 7,5, 5, 2,5 und 1 Gewichtsteilen pro Million Teile Nährlösung zugesetzt. Jeweils 100 ml Nährlösungswirkstoffgemisch sowie nur Nährlösung (Kontrolle) werden in 300 ml Erlenmeyerkolben gegeben. Die Nährlösung wird vor dem Wirkstoffzusatz mit einer Suspension der Alge Chlorella vulgaris beimpft; die Zelldichte wird auf $10^6$ Zellen/ml Nährlösung eingestellt. Nach 14-tägiger Aufstellung der Versuchsansätze bei Raumtemperatur und Lichtzutritt wird die Wirksamkeit beurteilt.

Das Ergebnis des Versuches zeigt, daß beispielsweise die Verbindung 1 bei der Anwendung von 2,5 ppm eine gute Wirkung gegen Algen hat.

Anwendungsbeispiel 5

Einer gegenüber Mikroorganismen stark anfälligen wäßrigen Dispersion auf Basis eines Polyacrylesters wird der Wirkstoff 1, gelöst in Propylenglykol, in Mengen von 0,1, 0,05, 0,025, 0,01, 0,005, 0,0025 und 0,001 %, bezogen auf das Gewicht der Dispersion, zugesetzt. Danach werden die Versuchsansätze, jeweils 100 ml, mit einer Mikroorganismensuspension beimpft, die als Keime Staphylococcus aureus, Escherichia coli, Pseudomonas aeruginosa, Proteus vulgaris, Aspergillus niger, Penicillium funiculosum, Geotrichum candidans und Rhodotorula rubra enthält. Die Keimdichte in der beimpften Dispersion liegt zwischen $10^6$ und $10^7$ Keime/ml. Nach einer Bebrütungsdauer von 21 Tagen bei 25°C werden aus den Versuchsansätzen Proben für das Wachstum von Bakterien, Schimmel- und Hefepilzen geeignete Agar-Nährmedien übertragen und diese 3 bzw. 7 Tage bebrütet, um noch lebensfähige Keime nachzuweisen:

Dieser Versuch zeigt, daß bereits 0,005 % Wirkstoff 1 ausreichen, um eine wäßrige Polyacrylester-Dispersion gegenüber Mikroorganismenbefall zu konservieren.

## Patentansprüche

1. 1-Hydroxypyrazolderivat der Formel

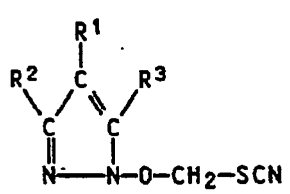

I

in der $R^1$, $R^2$ und $R^3$ unabhängig voneinander Wasserstoff oder Halogen bedeuten.

2. 4-Halogen-1-[(thiocyanato)-methoxy]-pyrazol.

3. 4-Chlor-1-[(thiocyanato)-methoxy]-pyrazol.

4. Verfahren zur Herstellung einer Verbindung gemäß Anspruch 1, dadurch gekennzeichnet, daß man ein 1-Hydroxypyrazol der Formel

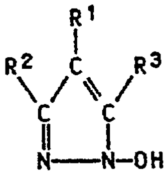

II,

in der $R^1$, $R^2$ und $R^3$ Wasserstoff oder Halogen bedeuten, mit einem Halogenmethylthiocyanat umsetzt.

5. Mikrozid enthaltend einen festen oder flüssigen Trägerstoff und ein 1-Hydroxipyrazolderivat der Formel

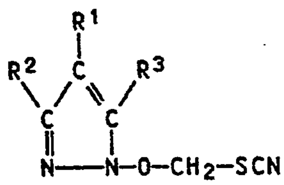

I

in der $R^1$, $R^2$ und $R^3$ unabhängig voneinander Wasserstoff oder Halogen bedeuten.

6. Verfahren zur Bekämpfung von Pilzen, Bakterien oder Algen im Materialschutz, dadurch gekennzeichnet, daß man die Mikroorganismen oder die vor dem Befall durch Mikroorganismen zu schützenden Gegenstände, Flüssigkeiten oder Suspensionen mit einem 1-Hidroxipirazolderivat der Formel

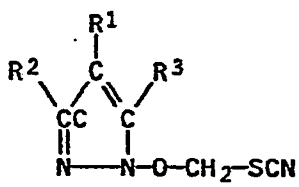

I

in der $R^1$, $R^2$ und $R^3$ unabhängig voneinander Wasserstoff oder Halogen bedeuten, behandelt.

## Claims

1. A 1-hydroxypyrazole derivative of the formula

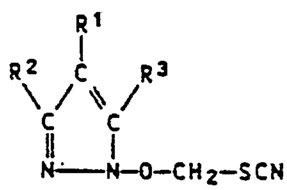

I

where $R^1$, $R^2$ and $R^3$ independently of one another are each hydrogen or halogen.

2. 4-Halo-1-[(thiocyanato)-methoxy]-pyrazole.

3. 4-chloro-1-[(thiocyanato)-methoxy]-pyrazole.

4. A process for the preparation of a compound as claimed in claim 1, wherein a 1-hydroxypyrazole of the formula

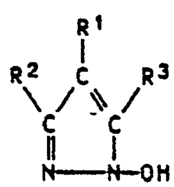

II

where $R^1$, $R^2$ and $R^3$ are each hydrogen or halogen, is reacted with a halomethyl thiocyanate.

5. A microbicide containing a solid or liquid carrier and a 1-hydroxypyrazole derivative of the formula

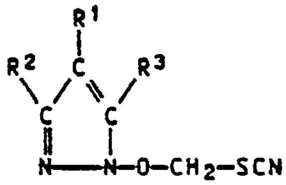

I

where $R^1$, $R^2$ and $R^3$ independently of one another are each hydrogen or halogen.

6. A method of controlling fungi, bacteria or algae in material protection, wherein the microorganisms, or the articles, liquids or suspensions to be protected from attack by microorganisms, are treated with a 1-hydroxypyrazole derivative of the formula

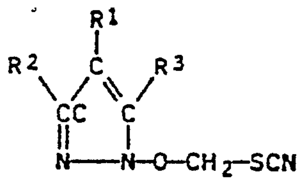

I

where $R^1$, $R^2$ and $R^3$ independently of one another are each hydrogen or halogen.

**Revendications**

1. Dérivé de 1-hydroxypyrazole de la formule

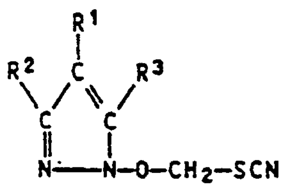

I

dans laquelle $R^1$, $R^2$ et $R^3$ représentent, indépendamment l'un de l'autre, hydrogène ou halogène.

2. 4-halogèno-1-(thiocyanato)-méthoxy-pyrazole.

3. 4-chloro-1-(thiocyanato)-méthoxy-pyrazole.

4. Procédé de préparation d'un composé selon la revendication 1, caractérisé par le fait qu'on fait réagir un 1-hydroxypyrazole de formule

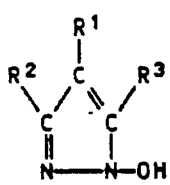

II,

dans laquelle $R^1$, $R^2$ et $R^3$ représentent hydrogène ou halogène avec un tyocyanate d'halogénométhyle.

5. Microcide contenant un support solide ou liquide et un dérivé de 1-hydroxypyrazole de la formule

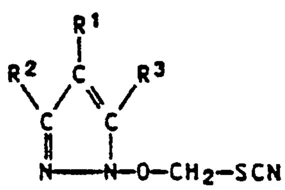

I

dans laquelle $R^1$, $R^2$ et $R^3$ représentent, indépendamment l'un de l'autre, hydrogène ou halogène.

6. Procédé de lutte contre les champignons, bactéries ou algues dans la protection des matériaux caractérisé par le fait qu'on traite les micro-organismes ou les objets, liquides ou suspensions à protéger avant l'attaque par des micro-organismes, avec un dérivé de 1-hydroxypyrazole de la formule

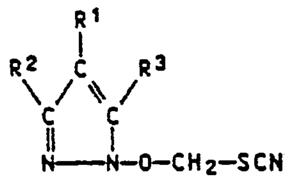

I

dans laquelle $R^1$, $R^2$ et $R^3$ représentent, indépendamment l'un de l'autre, hydrogène ou halogène.